# EUROPEAN PATENT SPECIFICATION

(11) **EP 2 211 710 B1**
(45) Date of publication and mention of the grant of the patent: **29.04.2015**
(21) Application number: 08855141.1
(22) Date of filing: 12.11.2008
(51) Int. Cl.: A61B 18/14, A61B 18/00, A61B 19/00

(54) **IRRIGATED ABLATION CATHETER HAVING MAGNETIC TIP FOR MAGNETIC FIELD CONTROL AND GUIDANCE**
BEWÄSSERTER ABLATIONSKATHETER MIT MAGNETISCHER SPITZE FÜR MAGNETFELDSTEUERUNG UND -FÜHRUNG
CATHÉTER D'ABLATION IRRIGUÉ AYANT UNE EXTRÉMITÉ MAGNÉTIQUE POUR LA COMMANDE ET LE GUIDAGE PAR CHAMP MAGNÉTIQUE

(30) Priority: 30.11.2007 US 948362; 10.12.2007 US 953615
(43) Date of publication of application: 04.08.2010
(73) Proprietor: St. Jude Medical, Atrial Fibrillation Division, Inc., St. Paul, Minnesota 55117-9913 (US)
(72) Inventor: KAUPHUSMAN, James, Champlin Minnesota 55311 (US); WANG, Huisun, Maple Grove Minnesota 55311 (US); DANDO, Jeremy D., Plymouth Minnesota 55447 (US); PURYEAR, Harry, Shoreview Minnesota 55126 (US)
(74) Representative: Kramer Barske Schmidtchen Patentanwälte PartG mbB
(86) International application number: PCT/US2008/083250
(87) International publication number: WO 2009/070448

(56) References cited:
- WO-A1-01/03589
- WO-A1-2005/048858
- WO-A1-2009/023385
- WO-A1-2009/152151
- US-A1- 5 061 823
- US-A1- 2004 267 106
- US-A1- 2007 270 791
- US-B2- 6 662 034
- US-B2- 6 977 469

## Description

### BACKGROUND OF THE INVENTION

### a. Field of the Invention

The present invention pertains generally to ablation catheters and electrode assemblies. More particularly, the present invention is directed toward ablation electrode assemblies for use in the human body having a magnetic tip for magnetic field control and guidance, a mechanism for irrigating targeted areas, and mapping characteristics.

### b. Background Art

Electrophysiology catheters are used for an ever-growing number of procedures. For example, catheters are used for diagnostic, therapeutic, and ablative procedures, to name just a few examples. Typically, the catheter is manipulated through the patient's vasculature and to the intended site, for example, a site within the patient's heart.

The catheter typically carries one or more electrodes, which may be used for ablation, diagnosis, or the like. There are a number of methods used for ablation of desired areas, including for example, radiofrequency (RF) ablation. RF ablation is accomplished by transmission of radiofrequency energy to a desired target area through an electrode assembly to ablate tissue at the target site.

Because RF ablation may generate significant heat, which if not carefully monitored and/or controlled can result in protein denaturation, blood coagulation, excess tissue damage, such as steam pop, tissue charring, and the like, it is desirable to monitor the temperature of the ablation assembly. It is further desirable to include a mechanism to irrigate certain target areas with biocompatible fluids, such as saline solution. This irrigation reduces or avoids excess, unwanted tissue damage, as well as blood coagulation and problems associated therewith. However, introduction of this irrigation solution may inhibit the ability to accurately monitor and/or control the temperature of the ablation assembly during use.

There are typically two classes of irrigated electrode catheters, open and closed irrigation catheters. Closed ablation catheters typically circulate a cooling fluid within the inner cavity of the electrode. Open ablation catheters, on the other hand, typically deliver the cooling fluid through open orifices on the electrode. Examples of these known catheters include the THERMOCOOL brand of catheters marketed and sold by Biosense-Webster. The current open irrigated ablation catheters use the inner cavity of the electrode, or distal member, as a manifold to distribute saline solution. The saline thus flows directly through the open orifices of the distal electrode member. This direct flow through the distal electrode tip lowers the temperature of the distal tip during operation, rendering accurate monitoring and control of the ablative process more difficult.

In these open electrode irrigated catheters, it has been determined that insulating the irrigation channels from the ablation electrode is beneficial. One such example was published on or around March 2005 in an article entitled "Saline-Irrigated Radiofrequency Ablation Electrode with Electrode Cooling," by Drs. Wittkampf and Nakagawa et al. Similarly, the PCT International Publication No. WO 05/048858, was published on June 2, 2005.

Recently, magnetic systems have been proposed, wherein magnetic fields produced by one or more electromagnets are used to guide and advance a magnetically tipped catheter. For example, U.S. Patent Application Publication No. 2007/0016006 discloses an apparatus and a method for guiding, steering, and advancing invasive devices and for accurately controlling their positions for providing positioning of magnetic fields and field gradient, for providing fields configured to push/pull, bend/rotate, and by further enabling apparatus to align the distal end of the catheter tip so as to achieve controlled movement in 3D space and ability of apparatus to control the magnetic field characteristics, preferably without excessively large power and field intensities that are potentially dangerous to medical personnel and that can be disruptive to other equipment.

US 2004/0267106 A1 describes an electrophysiology catheter. US 2007/0270791 A1 describes an ablation electrode assembly.

### BRIEF SUMMARY OF THE INVENTION

The invention provides an irrigated ablation electrode assembly according to claim 1. Preferred embodiments are defined in the dependent claims.

An Embodiments of the present invention provides an irrigated catheter configured to provide better electrode surface cooling and more accurate electrode tip temperature measurement, and having a magnetic tip that can be magnetically guided and controlled. The irrigated catheter may further include one or more monitoring or measuring electrodes for mapping or the like. The irrigation fluid is directed at target areas where coagulation is more likely to occur so as to minimize blood coagulation and the associated problems. The invention further provides for significant improvements over known irrigation catheters, including those disclosed by Wittkampf and Nakagawa et al., by providing a multiple piece irrigated ablation electrode assembly that has the advantages of irrigating the target area while simultaneously improving the operation, temperature response, temperature monitoring and/or control mechanisms of the ablation assembly, so as to prevent unwanted, unnecessary tissue damage and blood coagulation.

The present invention is directed to improved irrigated ablation electrode assemblies and methods useful in conjunction with irrigated catheter and pump assemblies and RF generator assemblies designed to monitor and control the ablation process while minimizing blood coagulation and unnecessary tissue damage, and with catheter guidance control and imaging systems designed to guide and control the magnetic tips of the electrode assemblies and perform mapping and other imaging functions.

In accordance with an aspect of the present invention, an irrigated ablation electrode assembly for use with an irrigated catheter device comprises at least one passageway for a fluid with an outlet disposed at an external surface of the electrode assembly; a permanent magnet; a shield separating the permanent magnet from the at least one passageway and from an exterior, the shield being substantially less oxidizable than the permanent magnet; and an electrode having an external electrode surface.

In some embodiments, the electrode forms at least a portion of the shield, and comprises an electrically conductive material that is substantially less oxidizable than the permanent magnet. The electrically conductive material is selected from the group consisting of platinum, gold, tantalum, iridium, stainless steel, palladium, and mixtures thereof, and the electrically conductive material is plated onto a substrate made of a biocompatible material that is substantially less oxidizable than the permanent magnet. The shield comprises one or more materials selected from the group consisting of silicone, polyimide, platinum, gold, tantalum, iridium, stainless steel, palladium, and mixtures thereof. In one example, the permanent magnet comprises NdFeB. At least one mapping electrode is spaced proximally from the electrode which is a distal electrode capable of ablation.

The electrode is disposed at a distal portion of the electrode assembly and includes an external electrode surface, and the electrode assembly further comprises a proximal portion which includes at least one proximal passageway for a fluid with an outlet disposed at an external surface of the proximal portion. The proximal portion comprises a material which is electrically nonconductive and has a lower thermal conductivity than a material of the electrode. The at least one proximal passageway extends toward the electrode at an acute angle with respect to the longitudinal axis of the proximal portion. The proximal portion comprises a material which is electrically nonconductive; the external surface of the proximal portion and the external electrode surface of the electrode at the distal portion meet at an intersection; and the at least one proximal passageway is configured to direct a fluid flow through the outlet toward a region adjacent the intersection. The permanent magnet is disposed in the distal portion, and the electrode assembly further comprises at least one temperature sensor disposed in the permanent magnet. The electrode includes an external electrode surface. The at least one electrode passageway is thermally insulated from the distal member by a poor thermal conductive material which is lower in thermal conductivity than a material of the electrode.

In some embodiments, which do not form a part of the invention, the permanent magnet comprises an annular permanent magnet with an axial opening to permit fluid flow to the at least one electrode passageway, and the electrode assembly further comprises a fluid lumen extending through the axial opening of the annular permanent magnet to the at least one electrode passageway. The fluid lumen comprises stainless steel braided polyimide forming a portion of the shield, and the electrode forms another portion of the shield. The shield includes a silicone seal to prevent fluid from reaching the annular permanent magnet via a junction between the electrode and the fluid lumen. The electrode is disposed at a distal portion of the electrode assembly, and the electrode assembly further comprises a proximal portion which includes at least one proximal passageway for a fluid with an outlet disposed at an external surface of the proximal portion. The proximal portion comprises a material which is electrically nonconductive. The external surface of the proximal portion and the external electrode surface of the electrode at the distal portion meet at an intersection. The at least one proximal passageway is configured to direct a fluid flow through the outlet toward a region adjacent the intersection.

In accordance with another aspect of this invention, a catheter comprises a shaft; and an irrigated ablation electrode assembly coupled to a distal end of the shaft. The irrigated ablation electrode assembly has at least one passageway for a fluid with an outlet disposed at an external surface of the electrode assembly; a permanent magnet; a shield separating the permanent magnet from the at least one passageway and from an exterior, the shield being substantially less oxidizable than the permanent magnet; and an electrode having an external electrode surface.

In some embodiments, the catheter further comprises a second permanent magnet disposed near the distal end of the shaft and spaced from the permanent magnet in the irrigated ablation electrode assembly.

The foregoing and other aspects, features, details, utilities, and advantages of the present invention will be apparent from reading the following description and claims, and from reviewing the accompanying drawings.

### BRIEF DESCRIPTION OF THE DRAWINGS

FIG. 1 is an isometric view of an ablation electrode assembly according to an embodiment of the present invention in conjunction with an irrigated catheter assembly operably connected to an RF generator assembly and a pump assembly.
FIG. 2 is an enlarged, isometric view of the ablation electrode assembly according to an embodiment of the present invention operably connected to an irrigated catheter assembly.
FIG. 3 is a cross-sectional view of the ablation electrode assembly of FIG. 2 taken along line 4-4 of FIG. 2.
FIG. 4 is a cross-sectional view of an ablation electrode assembly according to another embodiment, which does not form a part of the present invention.
FIG. 4A is a cross-sectional view of an ablation electrode assembly according to another embodiment, which does not form a part of the present invention.
FIG. 5 is a cross-sectional view of an ablation electrode assembly according to another embodiment, which does not form a part of the present invention.
FIG. 6 is a perspective view of the magnet structure of the Catheter Guidance Control and Imaging (CGCI) system.
FIG. 7A is a perspective view of the CGCI right section showing the hydraulically actuated core extended.
FIG. 7B is a perspective view of the CGCI right section showing the hydraulically actuated core extracted.
FIG. 7C is a system block diagram for a surgery system that includes an operator interface, a catheter guidance system, and surgical equipment.
FIG. 7D is a block diagram of the imaging module for use in a CGCI surgery procedure that includes the catheter guidance system, a radar system, Hall Effect sensors, and a hydraulically actuating core extension mechanism.
FIG. 8A is a first perspective view of a catheter assembly.
FIG. 8B is a second perspective view of the catheter assembly.
FIG. 9A is a side view of the apparatus of FIG. 6.
FIG. 9B is an underside view of the apparatus of FIG. 6.
FIG. 10 is an isometric view showing the apparatus of FIG. 6 in an open mode where the left and right clusters are separated.
FIG. 11 is a side view of the configuration shown in FIG. 10.
FIG. 12 is an underside view of the configuration shown in FIG. 10.
FIG. 13 is an end view of the configuration shown in FIG. 10.
FIG. 14 is a block diagram of one embodiment of the CGCI apparatus with magnetic sensors.

### DETAILED DESCRIPTION OF EXEMPLARY EMBODIMENTS

### Irrigated Catheter with Magnetic Tip

In general, the instant invention relates to irrigated ablation electrode assemblies, and to methods of manufacturing and using such irrigated ablation electrode assemblies. For purposes of this description, similar aspects among the various embodiments described herein will be referred to by the same reference number. As will be appreciated, however, the structure of the various aspects may be different among the various embodiments.

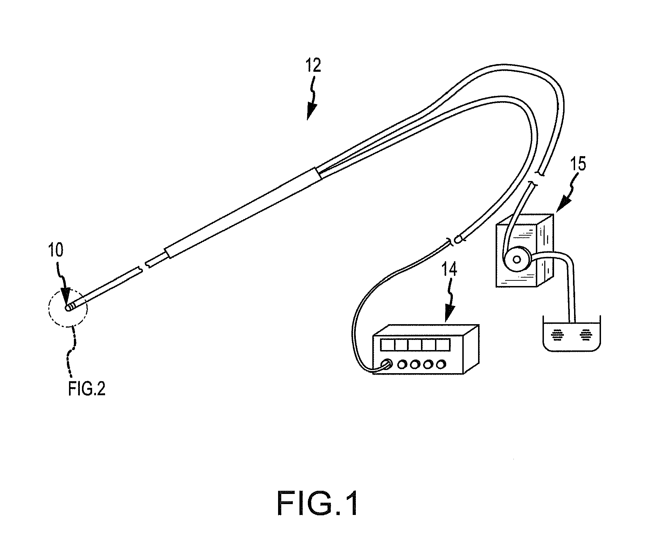

As seen in FIG. 1, the ablation electrode assembly may comprise part of an irrigated ablation catheter assembly 12, operably connected to a pump assembly 15 and an RF generator assembly 14 which serves to facilitate the operation of ablation procedures through monitoring any number of chosen variables (e.g., temperature of the ablation electrode, ablation energy, and position of the assembly), assist in manipulation of the assembly during use, and provide the requisite energy source delivered to the electrode assembly 10. The present embodiments describe RF ablation electrode assemblies and methods, but it is contemplated that the present invention is equally applicable to any number of other ablation electrode assemblies where the temperature of the device and the targeted tissue areas is a factor during the procedure.

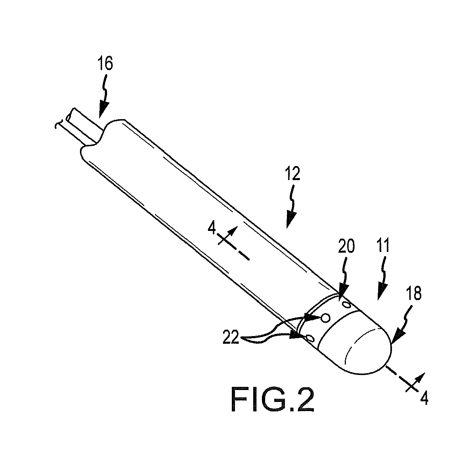

FIG. 1 is a general perspective view of an irrigated ablation catheter assembly having an RF generator assembly 14 and a fluid pump assembly 15 operably connected to an irrigated catheter assembly 12 having an irrigated electrode assembly 10 according to the present invention operably attached thereto. The structural and functional features of the catheter assembly 12 and the RF generator assembly 14 and pump assembly 15 are well-known to those of skill in the art. For example, the RF generator assembly could be an IBI-1500T RF Cardiac Ablation Generator available from Irvine Biomedical, Inc. in Irvine, California 92614. The RF generator assembly could also be any other known assembly, including, for example, a Stockert RF generator available from Biosense, or one of the Atakr® series of RF generators available from Medtronic. The pump assembly can be any known assembly, including fixed volume rolling pumps, variable volume syringe pumps, and any other pump assembly known to those of skill in the art. FIGS. 2-5, discussed in more detail below, exemplify various embodiments of the irrigated ablation electrode assembly 10.

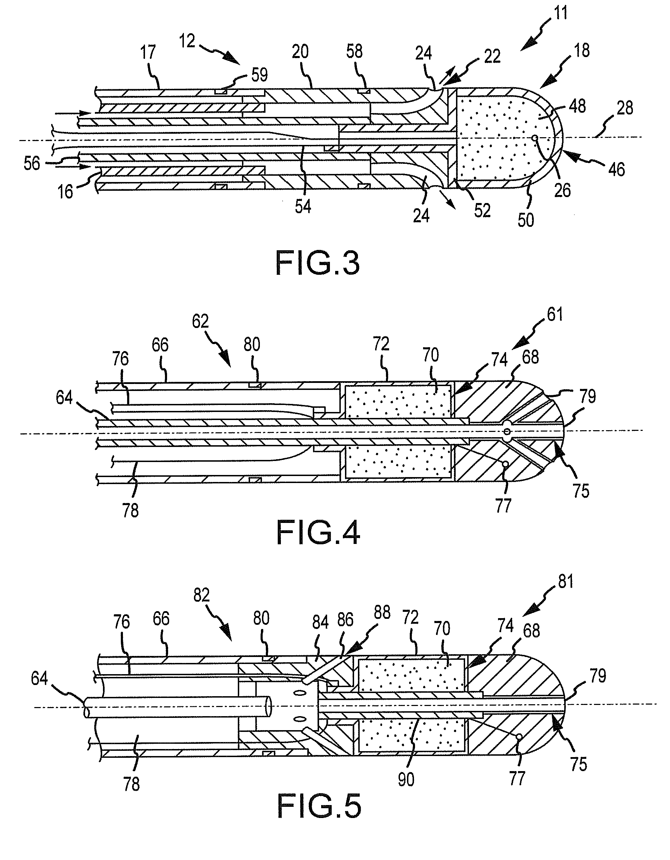

FIG. 2 is an isometric view of an ablation electrode assembly 11 connected to an irrigated ablation catheter assembly 12 having a fluid delivery tube 16 therein. The ablation electrode assembly 11 generally comprises an irrigation member 20 and an ablation electrode member 18. The orientation of the members 18, 20 are generally such that the ablation electrode assembly 18 is situated at the distal end of the assembly with the irrigation member 20 located at the proximal end of the assembly, although it is conceivable the orientation could be reversed. The proximal member 20 has at least one passageway 24 (see FIG. 3) and at least one outlet 22 for delivery of a fluid to targeted tissue areas and the outside of the electrode assembly 11. The distal member 18 further comprises at least one temperature sensing mechanism 26 (see FIG. 3) disposed therein and operably connected to the RF generator assembly 14. The distal member 18 is comprised of any electrically, and potentially thermally, conductive material known to those of ordinary skill in the art for delivery of ablative energy to target tissue areas. Examples of the electrically conductive material include gold, platinum, iridium, palladium, tantalum, stainless steel, and any mixtures thereof. Moreover, there are a number of electrode designs contemplated within the scope of the present invention including tip electrodes, ring electrodes, and any combination thereof.

In general accordance with the embodiments described herein, the fluid passageway(s) 24 and outlet(s) 22 are separated from the distal member 18, and accordingly the temperature sensing mechanism 26, by at least one poor thermally conductive material. A poor thermally conductive material is one with physical attributes that decrease heat transfer between the passageway(s) 24 and the distal member 18 by about 10% or more, and more preferably by about 25% or more measured by known methods to one of ordinary skill in the art. In particular embodiments, materials that decreased heat transfer by more than approximately 75% performed favorably. It is further contemplated that a poor thermally conductive material could have physical attributes that decrease heat transfer less than about 10%, provided that the remaining structural components are selected with the appropriate characteristics and sensitivities to maintain adequate monitoring and control of the process. Thus, while these properties are preferred, the poor thermally conductive material may be any material known to one of skill in the art consistent with the scope of the invention. Examples of poor thermally conductive materials useful in conjunction with the present invention include, but are not limited to, high-density polyethylene (HDPE), polyimides, polyaryletherketones, polyetheretherketones, polyurethane, polypropylene, oriented polypropylene, polyethylene, crystallized polyethylene terephthalate, polyethylene terephthalate, polyester, ceramics, and plastics such as acetal, and mixtures thereof.

As shown in more detail with respect to specific embodiments below, the poor thermally conductive material may be the material comprising the proximal member 20, or the distal member 18, a separate material from the proximal member 20 and the distal member 18, or any combination thereof. Additionally, the passageway(s) 24 and outlet(s) 22 defined by the proximal member 18 may also be separated longitudinally from the end 46 (see FIG. 3) of the distal member 18 thereby providing the benefit of insulating the passageway(s) 24 from the temperature sensor(s) 26 for improved temperature monitoring of the ablated target area during operation. The poor thermally conductive material, and the separation from the temperature sensing mechanism 26 disposed near the end 46 of the distal member 18, serve individually, and cooperatively, to minimize the effect of the lower temperature of the fluid delivered through the passageway(s) 24 and outlet(s) 22 from the temperature sensing mechanism(s) 26 within the distal member 18. The separation of the passageway(s) 24 and outlet(s) 22 from the distal member 18, and more particularly the temperature sensing mechanism 26, facilitates the dual purposes of (1) effectively irrigating the electrode assembly 11 and the targeted tissue area to minimize coagulation and unwanted tissue damage and (2) effectively controlling the operation of the ablation electrode assembly 11 in accordance with objects of the present invention.

FIG. 3 is a cross-sectional view of an embodiment of the ablation electrode assembly 11. An ablation electrode assembly 11 is connected to an irrigated catheter assembly 12 having a fluid delivery tube 16 and a catheter shaft 17. The ablation electrode assembly 11 comprises a proximal member or manifold 20, a distal member 18, and a temperature sensing mechanism 26 operably connected to the RF generator assembly 14 (see FIG. 1). In this embodiment, the proximal member 20 itself is comprised of a poor thermally conducting material that serves to insulate the irrigation fluid from the remaining portions of the assembly 11. Preferably the proximal member 20 is made from a poor thermally conductive polymer, more preferably from a polyether ether ketone ("PEEK") because of this material's combination of thermal and physical properties. Another possible material is Ultem® polyetherimide. The proximal member 20 is configured to receive the fluid tube 16 of the catheter assembly 12 and comprises a plurality of passageways 24 (e.g., 4-8 passageways) extending from a central axis 28 of the assembly 11 axially toward the outer portion of the proximal member 20 terminating in corresponding outlets 22. Preferably, the plurality of passageways 24 are equally distributed around the proximal member 20 so as to provide equal distribution of fluid to the targeted tissue area and the outside of the assembly 11. The passageway 24 may be a single, annular passageway, or a number of individual passageways equally distributed around the proximal member 20. In this embodiment, the passageways 24 are at an acute angle with respect to the longitudinal axis 28 of the assembly 11. In operation, fluid is pumped through the delivery tube 16 and passes through the passageways 24 and through the outlets 22 where it contacts with targeted tissue areas and the outside portion of the ablation electrode assembly 11.

In this embodiment, the fluid delivery conduits or passageways 24 extend at an angle substantially less than perpendicular to the longitudinal axis 28. Angling of the passageways 24 away from perpendicular, but less than parallel, further assists in the delivery of the fluid to the targeted tissue areas, further decreases the risk of coagulation of the bodily fluids during ablation procedures, and allows for improved measurement and control of the ablation assembly 11 during operation. More specifically, the passageways 24 are oriented to direct irrigation fluid flow at the target area adjacent, preferably immediately adjacent, the intersection between the proximal member 20 and the distal member 18. Blood coagulation is more likely to occur in the target area due to a sharp rise in RF intensity, material discontinuity, and potentially geometric discontinuity caused by manufacturing imperfection in joining the proximal member 20 and the distal member 18. In specific embodiments, the passageways 24 extend at an angle between approximately 20 and 70 degrees, preferably at an angle between approximately 30 and 60 degrees, and more preferably at an angle of approximately 30 degrees. It is also contemplated that the passageways may be further angled in a second dimension, such that the passageways and orifices are configured to provide fluid to the external portion of the assembly in a swirling, or helical fashion. This configuration also serves to keep the fluid in closer proximity to the electrode assembly, thereby further preventing against coagulation during operation.

The distal member 18 of the ablation electrode assembly 11 has a generally cylindrical shape terminating in a rounded end which may be a hemispherical end or an end that is non-spherical. The distal member 18 includes a permanent magnet 48 at least partially encased in a distal electrode shell 50 and an electrode anchor 52. The permanent magnet 48 is desirably made of NdFeB which has a strong magnetic field so that only one such permanent magnet is needed for magnetic field control and guidance of the catheter tip (instead of a plurality of magnets spaced apart from each other). Other rare earth permanent magnets with similar characteristics may be used in other embodiments. If two or more permanent magnets are used, additional materials may be considered. The permanent magnet 48 typically has a length of about 2-6 mm, typically about 4 mm, in the longitudinal direction. The distal electrode shell 50 provides most of the external surface of the distal electrode. The electrode anchor 52 is coupled to the proximal member 20 and connected to a power line or cable such as an RF wire 54. The electrode anchor 52 may be connected to the proximal member 20 by any known mechanism including adhesives, press-fit configurations, snap-fit configurations, or the like. An inner tube 56 is connected to the electrode anchor 52 and/or the proximal member 20 to accommodate the power line 54 and the temperature sensor conductor for the temperature sensor 26. Because the temperature sensor 26 is embedded in the permanent magnet 48, the permanent magnet material preferably is a good thermal conductor (e.g., NdFeB) so that the temperature sensor 26 can measure the temperature of the distal electrode accurately.

In the embodiment shown, the distal electrode shell 50, the electrode anchor 52, and the inner tube 56 form a shield that keeps the permanent magnet 48 from exposure to irrigation and/or bodily fluids, comprising an inner shield that separates the permanent magnet 48 from the irrigation fluid including the passageways 24 and an outer shield that separates the permanent magnet 48 from the exterior. Because the permanent magnet 48 is highly oxidizable, any contact between the permanent magnet and liquid is undesirable since oxidation of the permanent magnet 48 can lead to corrosion problems. The shield prevents such contact from occurring. The materials for the shield are less oxidizable, preferably substantially less oxidizable, than the permanent magnet 48. For instance, the oxidization rate of a shield material is less than about 50%, more preferably less than about 20%, most preferably less than about 5%, of the oxidation rate of the permanent magnet 48. The distal electrode shell 50 and the electrode anchor 52 are made of an electrically conductive material such as platinum, gold, tantalum, iridium, stainless steel, palladium, tantalum, and mixtures thereof. The electrically conductive material selected is preferably biocompatible. In some embodiments, the biocompatible electrically conductive material is plated onto a substrate made of copper or beryllium copper to improve the biocompatibility of the distal electrode shell 50 and the electrode anchor 52. The electrode anchor 52 may be laser welded to the distal electrode shell 50. The inner tube 56 may be made of silicone, polyimide, stainless steel braided polyimide, or the like. The inner tube 56 may be thermally bonded or molded onto the platinum anchor 52. In alternate embodiments, the distal electrode shell 50 and the electrode anchor 52 form a complete shield around the permanent magnet 48 without the need for the inner tube 56 for separating the permanent magnet 48 from irrigation fluid flow.

The proximal member 20 preferably is made of a poor thermally conductive material (as discussed above) having a thermal conductivity that is lower, more preferably substantially lower, than the thermal conductivity of the material of the distal member 18. The proximal passageways 24 do not come into contact with any interior portion of the distal member 18. In this way, the irrigation fluid flowing through the proximal passageways 24 is substantially insulated from the electrode and the temperature sensor of the distal member 18 by distance and material of poor conductivity, so that the temperature sensor 26 can more accurately measure the temperature of the distal electrode. The proximal members may be made of a variety of materials that have insulating properties such as, for example, acetal, polyetheretherketone (PEEK), and high-density polyethylene (HDPE), as well as other materials of poor thermal conductivity mentioned above.

One or more monitoring or measuring electrodes may be provided in the catheter assembly 12 for mapping or other monitoring or measuring functions. FIG. 3 shows two monitoring electrodes 58, 59 that are ring electrodes spaced from the distal electrode 18. To facilitate catheter tip positioning and location in a mapping system, the position of each electrode is determined. Calibration of the positioning system is achieved by the two monitoring electrodes 58, 59 separated by a known interelectrode distance, or by the distal electrode 18 and one monitoring electrode (58 or 59) that are separated by a predetermined distance. In use, a voltage is sensed between one electrode on the catheter assembly 12 (typically the distal electrode 18) and a reference electrode on the patient's body (suitably a surface electrode on the patient's skin). For a catheterization procedure which is to lead to ablation, sensing is performed to gather data relating to the heart, such as the location of an arrhythmia focus. Such data gathering techniques are well known in the art. The location information is determined based on the calibration (see, e.g., U.S. Patent Nos. 5,697,377 and 5,983,126), and the sensed information and location are stored and/or mapped.

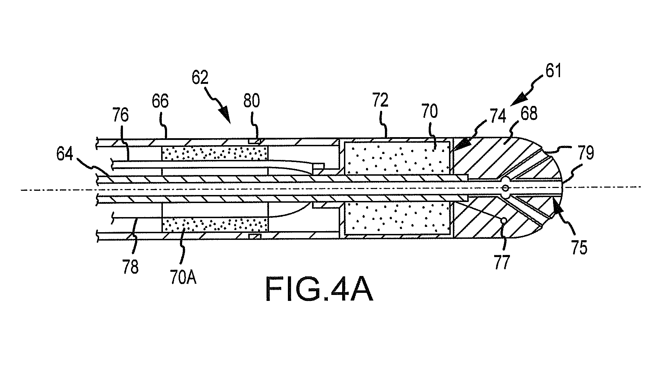

FIG. 4 is a cross-sectional view of another embodiment or the ablation electrode assembly 61, which does not form part of the present invention . The ablation electrode assembly 61 is connected to an irrigated catheter assembly 62 having a fluid delivery tube or lumen 64 and a catheter shaft 66. The ablation electrode assembly 61 comprises a distal member 68, a permanent magnet 70 disposed proximal to the distal member 68, and a shell 72 surrounding the outer surface and the proximal surface of the permanent magnet 70. The distal member 68 has a generally cylindrical shape terminating in a rounded end which may be a hemispherical end or an end that is non-spherical. The permanent magnet 70 is an annular member having an inner surface covered by a portion of the fluid delivery tube 64. The permanent magnet 70 is desirably made of NdFeB which has a strong magnetic field so that only one such permanent magnet is needed for magnetic field control and guidance of the catheter tip (instead of a plurality of magnets spaced apart from each other). The permanent magnet 48 typically has a length of about 2-6 mm, typically about 4 mm in the longitudinal direction. The distal member 68, shell 72, and fluid delivery tube 64 form a shield that keeps the permanent magnet 70 from exposure to liquid, comprising an inner shield that separates the permanent magnet 70 from the irrigation fluid flowing through the catheter assembly 62 and an outer shield that separates the permanent magnet 70 from the exterior. A sealant 74 is preferably provided between the proximal surface of the distal member 68 and the distal surface of the permanent magnet 70 to further ensure no liquid reaches the permanent magnet 70 via the junction between the distal member 68 and the fluid delivery tube 64.

The distal member 68 provides the external surface of the distal electrode. The shell 72 may also be an electrically conductive surface to provide additional external surface of the distal electrode. In that case, the electrode shell 72 is connected to a power cable or line such as an RF wire 76. One or more temperature sensors 77 may be provided in the distal member 68 and the temperature sensor conductor 78 for the temperature sensor 77 extends proximally through the catheter shaft 66.

Because the permanent magnet 70 is highly oxidizable, any contact between the permanent magnet and liquid is undesirable. The shield prevents such contact from occurring. The materials for the shield are less oxidizable, preferably substantially less oxidizable, than the permanent magnet 70. The distal member 68 and the electrode shell 72 are made of an electrically conductive material such as platinum, gold, tantalum, iridium, stainless steel, palladium, tantalum, and mixtures thereof. The electrically conductive material selected is preferably biocompatible. In some embodiments, the biocompatible electrically conductive material is plated onto a substrate made of copper or beryllium copper to improve the biocompatibility of the distal member 68 and the electrode shell 72. The fluid delivery tube 64 is electrically nonconductive, and may be made of silicone, polyimide, stainless steel braided polyimide, or the like. The electrode shell 72 is connected to the distal member 68 by laser weld or the like. The distal member 68 and the electrode shell 72 form the distal electrode. The shell 72 may be connected to the catheter shaft 66 using adhesives or the like. The fluid delivery tube 64 may be connected to the shell 72, permanent magnet 70, and distal member 68 by thermal bonding, molding, adhesives, or the like.

The fluid delivery tube 64 flows fluid through one or more distal passageways 79 in the distal member 68 to their external outlets. There is preferably a central passageway along the longitudinal axis of the distal member 68 and, optionally, additional passageways distributed around the central passageway. The passageways 79 are preferably lined with a poor thermally conducting material 75 such as a polyether ether ketone ("PEEK") that serves to insulate the fluid from the material of the distal member 68 and from the temperature sensor 77. In this way, the fluid flow through the passageways 79 does not influence the measurement of the temperature sensor 77, so that the temperature sensor 77 can more accurately measure the temperature of the distal electrode. Preferably, the additional passageways are equally distributed around the central passageway so as to provide equal distribution of fluid to the targeted tissue area and the outside of the assembly 61.

One or more monitoring or measuring electrodes may be provided in the catheter assembly 62 for mapping or other monitoring or measuring functions. FIG. 4 shows one monitoring electrode 80 that is a ring electrode spaced from the distal electrode (formed by the distal member 68 and the electrode shell 72) by a known interelectrode distance for calibration. In use, a voltage is sensed between the distal electrode (68 and 72) and a reference electrode on the patient's body (suitably a surface electrode on the patient's skin). The location information is determined based on the calibration, and the sensed information and location are stored and/or mapped.

FIG. 4A shows an irrigated catheter assembly 62A that is virtually identical to the irrigated catheter assembly 62 of FIG. 4. The assembly 62A includes a second permanent magnet 70A disposed near the distal end of the shaft 66 and spaced from the first permanent magnet 70. In the embodiment shown, which does not form part of the present invention , the second permanent magnet 70A is an annular magnet and is smaller in size and thickness than the first permanent magnet 70. The second permanent magnet 70A does not require an additional shield because it is disposed in a space between the catheter shaft 66 and the fluid delivery tube 64 which is free from exposure to liquid. Of course, the second permanent magnet may have other configurations in different embodiments, and may be formed in the irrigated ablation electrode assembly 61 instead of being inside the catheter shaft 66 and spaced proximally from the electrode assembly 61. Additional permanent magnets in the assembly may provide additional options for magnetically controlling and guiding the catheter tip.

FIG. 5 is a cross-sectional view of another embodiment of the ablation electrode assembly 81, which does not form part of the present invention and which is connected to an irrigated catheter assembly 82. The electrode assembly 81 of FIG. 5 is similar to the electrode assembly 61 of FIG. 4, in that it also includes a distal member 68, a permanent magnet 70, a shell 72 connected to an RF wire 76, a sealant 74, and a temperature sensor 77 connected to a temperature sensor conductor 78. In this embodiment, the distal member 68 has a central passageway 79 that is preferably lined with a poor thermally conducting material 75 such as a polyether ether ketone ("PEEK"). A fluid delivery tube 64 extends through a catheter shaft 66 to the electrode assembly 81. One or more monitoring or measuring electrodes 80 may be provided in the catheter assembly 62 for mapping or other monitoring or measuring functions.

In FIG. 5, the ablation electrode assembly 81 includes a proximal member 84 located on the proximal side of the permanent magnet 70 and electrode shell 72. The proximal member 84 has at least one proximal passageway 86 with at least one outlet 88 for delivery of a fluid to targeted tissue areas and the outside of the electrode assembly 81. The proximal passageway(s) 86 and outlet(s) 88 are separated from the distal member 68 and electrode shell 72, and accordingly the temperature sensing mechanism 77, by at least one poor thermally conductive material. The poor thermally conductive material may be the material comprising the proximal member 84, or the distal member 68, a separate material from the proximal member 84 and the distal member 68, or any combination thereof. In this embodiment, the proximal member 84 is comprised of a poor thermally conducting material that serves to insulate the fluid from the remaining portions of the assembly 81. The proximal member 84 is configured to receive the fluid tube 64 of the catheter assembly 82 and comprises a plurality of proximal passageways 86 (e.g., 4-8 passageways) extending from a central axis of the assembly 81 axially toward the outer portion of the proximal member 84 terminating in corresponding outlets 88. Preferably, the plurality of proximal passageways 86 are equally distributed around the proximal member 84 so as to provide equal distribution of fluid to the targeted tissue area and the outside of the assembly 81. The proximal passageway 86 may be a single, annular passageway, or a number of individual passageways equally distributed around the proximal member 84. In this embodiment, the proximal passageways 86 are at an acute angle with respect to the longitudinal axis of the assembly 81. In operation, fluid is pumped through the delivery tube 64 and passes through the proximal passageways 86 and through the outlets 88 where it contacts with targeted tissue areas and the outside portion of the ablation electrode assembly 81.

In this embodiment, the proximal passageways 86 extend at an angle substantially less than perpendicular to the longitudinal axis. Angling of the passageways 86 away from perpendicular, but less than parallel, further assists in the delivery of the fluid to the targeted tissue areas, further decreases the risk of coagulation of the bodily fluids during ablation procedures, and allows for improved measurement and control of the ablation assembly 81 during operation. More specifically, the proximal passageways 86 are oriented to direct irrigation fluid flow at the target area adjacent, preferably immediately adjacent, the intersection between the proximal member 84 and the electrode shell 72. Blood coagulation is more likely to occur in the target area due to a sharp rise in RF intensity, material discontinuity, and potentially geometric discontinuity caused by manufacturing imperfection in joining the proximal member 84 and the electrode shell 72. In specific embodiments, the proximal passageways 24 extend at an angle between approximately 20 and 70 degrees, preferably at an angle between approximately 30 and 60 degrees, and more preferably at an angle of approximately 30 degrees. It is also contemplated that the proximal passageways may be further angled in a second dimension, such that the proximal passageways and orifices are configured to provide fluid to the external portion of the assembly in a swirling, or helical fashion. This configuration also serves to keep the fluid in closer proximity to the electrode assembly, thereby further preventing against coagulation during operation.

The proximal member 84 further includes a longitudinal outlet that transfers fluid through a central conduit 90 to the central passageway 79 of the distal member 68. The central conduit 90 is electrically nonconductive, and may be made of silicone, polyimide, stainless steel braided polyimide, or the like. The central conduit 90 may be connected to the electrode shell 72, permanent magnet 70, and distal member 68 by thermal bonding, molding, adhesives, or the like. The distal member 68, electrode shell 72, and central conduit 90 form a shield that separates the permanent magnet 70 from the irrigation fluid and the exterior.

### Catheter Guidance Control and Imaging (CGCI)

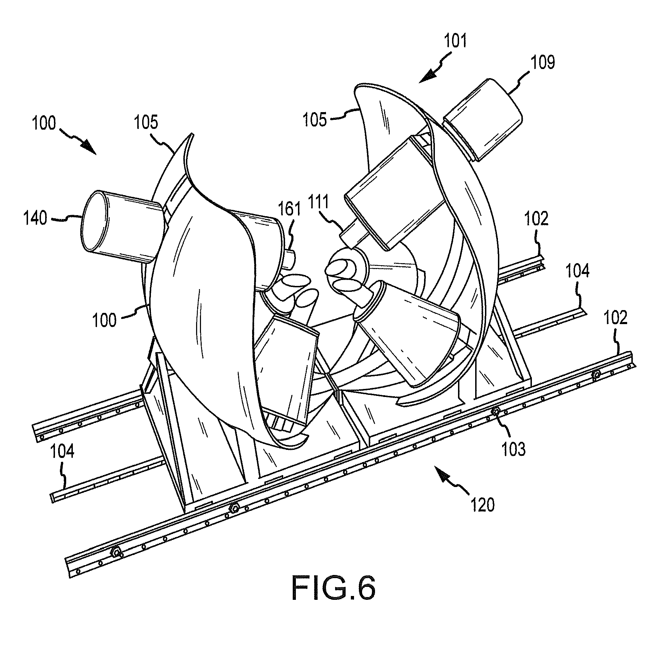

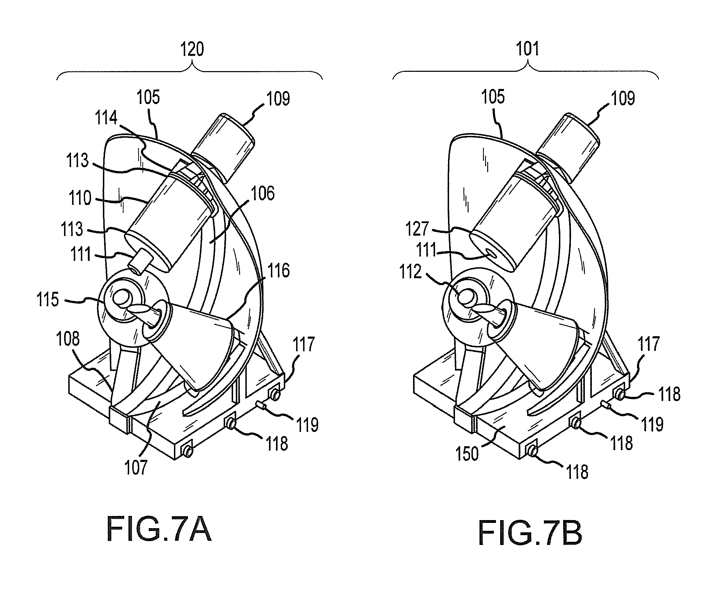

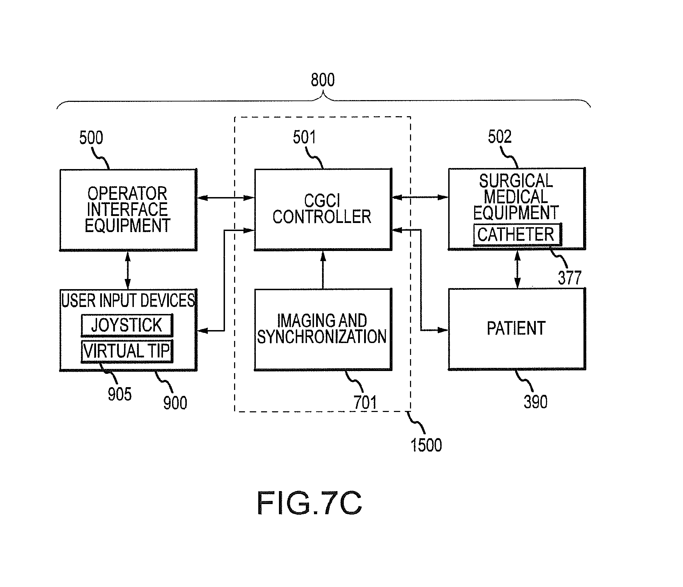

One example of a system for magnetically guiding and controlling a catheter having a magnetic tip is found in U.S. Patent Application Publication No. 2007/00160 06. FIGS. 6, 7A, and 7B are isometric drawings of a Catheter Guidance Control and Imaging (CGCI) system 1500 (FIG. 7C), having a left coil cluster 100 and a right coil cluster 101 provided to rails 102. The rails 102 act as guide alignment devices. The CGCI system workstation 1500 includes a structural support assembly 120, a hydraulic system 140, and a propulsion system 150.

A central arc 106 supports an upper cylindrical coil 110 and two shorter arcs 107, 108 support two conical shaped coils 115, 116. The two shorter arcs 107, 108 are displaced from the central arc 106 by approximately 35 degrees. The angle of separation between the two smaller arcs is approximately 70 degrees. At the end of each arc 106, 107 and 108 is a machined block of 1010 steel with a connection that provides for attachment of the coil assemblies 115, 116, 110.

Two curved shield plates 105 form a shield to at least partially contain and shape the magnetic fields. The shields 105 also provide lateral strength to the assembly. A base 117 houses the propulsion system 150 and locking mechanism 118. In one embodiment, the plates 105 are made from steel, nickel, or other magnetic material.

In addition to FIG. 6, FIGS. 7A and 7B further show various mechanical details which form the CGCI cluster half section (right electromagnetic cluster 101). A locking hole 103, a spur-drive rail 104, cam rollers 118, and the solenoid locking pin 119, are configured to allow portions of the CGCI to move along the tracks 102. The cluster 101 includes three electromagnets forming a magnetic circuit. The left coil 116 and right coil 115 are mounted as shown and are supported by C-Arms 107 and 108. The coil 110 includes a hydraulically actuated core 111, supported by a coil clamping disc 127 made of stainless steel. A coil stress relief disc 113 is made of Teflon. The coil cylinder 110, is enclosed by a coil base disc 114 made of stainless steel. The coil core 111 is actuated (extended and retracted) by a hydraulic system 109. FIG. 7B shows the right coil cluster 101 with the hydraulically actuated core 111 retracted by the use of the hydraulic system 109 which allows the CGCI to shape the magnetic field.

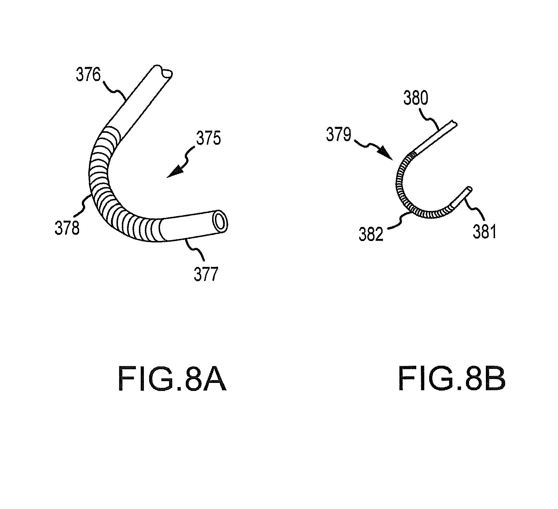

FIG. 7C is a system block diagram for a surgery system 800 that includes an operator interface 500, the CGCI system 1500, surgical equipment 502 (e.g., a catheter tip 11 in FIG. 3, a catheter tip 61 in FIG. 4, a catheter tip 81 in FIG. 5, or a catheter tip 377 in FIG. 8A, etc.), one or more user input devices 900, and a patient 390. The user input devices 900 can include one or more of a joystick, a mouse, a keyboard, a virtual tip 905, and other devices to allow the surgeon to provide command inputs to control the motion and orientation of the catheter tip 377 (or tip 11, 61, 81).

In one embodiment, the CGCI system 1500 includes a controller 501 and an imaging synchronization module 701. FIG. 7C shows the overall relationship between the various functional units and the operator interface 500, auxiliary equipment 502, and the patient 390. In one embodiment, the CGCI system controller 501 calculates the Actual Tip (AT) position of the distal end of a catheter. Using data from the Virtual Tip (VT) 905 and the imaging and synchronization module 701, the CGCI system controller 501 determines the position error, which is the difference between actual tip position (AP) and the desired tip position (DP). In one embodiment, the controller 501 controls electromagnets to move the catheter tip in a direction selected to minimize the position error (PE). In one embodiment, the CGCI system controller 501 provides tactile feedback to the operator by providing force-feedback to the VT 905.

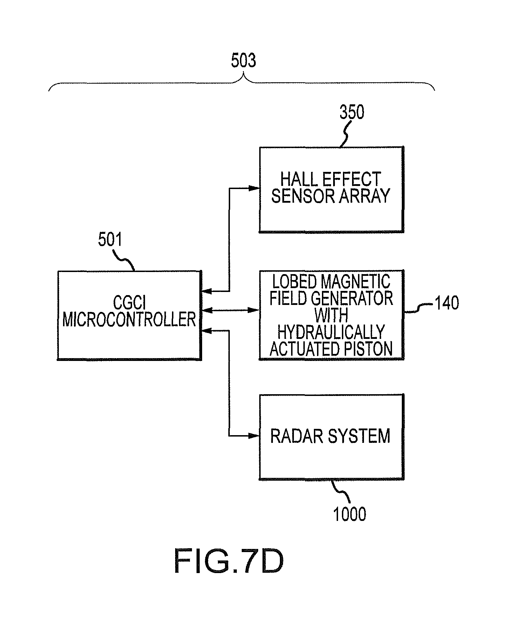

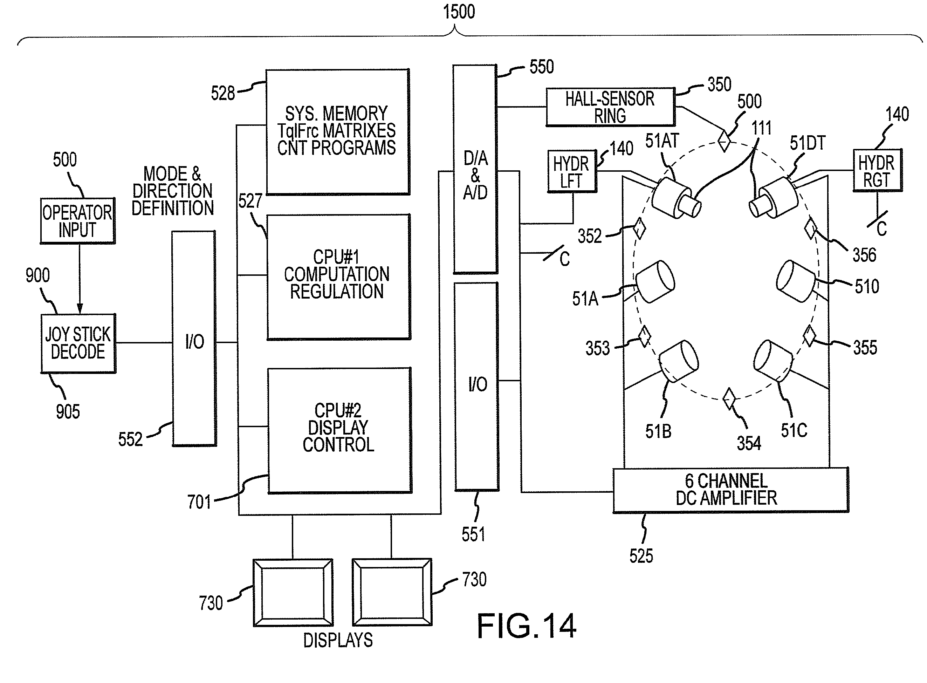

FIG. 7D is a block diagram of a surgery system 503 that represents one embodiment of the CGCI system 1500. The system 503 includes the controller 501, a radar system 1000, a Hall effect sensor array 350, and the hydraulically actuated mechanism 140. In one embodiment, the sensor 350 includes one or more Hall effect magnetic sensors. The radar system 1000 can be configured as an ultra-wideband radar, an impulse radar, a Continuous-Wave (CW) radar, a Frequency-Modulated CW (FM-CW) radar, a pulse-Doppler radar, etc. In one embodiment, the radar system 1000 uses Synthetic Aperture Radar (SAR) processing to produce a radar image. In one embodiment, the radar system 1000 includes an ultra-wideband radar such as described, for example, in U.S. Pat. No. 5,774,091. In one embodiment, the radar 1000 is configured as a radar range finder to identify the location of the catheter tip 377. The radar 1000 is configured to locate reference markers (fiduciary markers) placed on the patient 390. Data regarding location of the reference markers can be used, for example, for image capture synchronization 701. The motorized hydraulically and actuated motion control mechanism 140 allows the electromagnets of the cylindrical coils 51AT and 51DT (see FIG. 14) to be moved relative to the patient 390.

In one embodiment, the use of the radar for identifying the position of the catheter tip 377 has advantages over the use of Fluoroscopy, Ultrasound, Magnetostrictive sensors, or SQUID. Radar can provide accurate dynamic position information, which provides for real-time, relatively high resolution, relatively high fidelity compatibility in the presence of strong magnetic fields. Self-calibration of the range measurement can be based on time-of-flight and/or Doppler processing. Radar further provides for measurement of catheter position while ignoring "Hard" surfaces such as a rib cage, bone structure, etc., as these do not interfere with measurement or hamper accuracy of the measurement. In addition, movement and displacement of organs (e.g., pulmonary expansion and rib cage displacement as well as cardio output during diastole or systole) do not require an adjustment or correction of the radar signal. Radar can be used in the presence of movement since radar burst emission above 1 GHz can be used with sampling rates of 50Hz or more, while heart movement and catheter dynamics occur at 0.1 Hz to 2Hz.

In one embodiment, the use of the radar 1000 reduces the need for complex image capture techniques normally associated with expensive modalities such as fluoroscopy, ultrasound, Magnetostrictive technology, or SQUID which require computationally intensive processing in order to translate the pictorial view and reduce it to a coordinate data set. Position data synchronization of the catheter tip 377 and the organ in motion is readily available through the use of the radar 1000. The radar 1000 can be used with phased-array or Synthetic Aperture processing to develop detailed images of the catheter location in the body and the structures of the body. In one embodiment, the radar system includes an Ultra Wide Band (UWB) radar with a relatively high resolution swept range gate. In one embodiment, a differential sampling receiver is used to effectively reduce ringing and other aberrations included in the receiver by the near proximity of the transmit antenna. As with X-ray systems, the radar system can detect the presence of obstacles or objects located behind barriers such as bone structures. The presence of different substances with different dielectric constants such as fat tissue, muscle tissue, water, etc., can be detected and discerned. The outputs from the radar can be correlated with similar units such as multiple catheters used in electrophysiology (EP) studies while detecting spatial location of other catheters present in the heart lumen. The radar system 1000 can use a phased array antenna and/or SAR to produce 3D synthetic radar images of the body structures, catheter tip and organs.

In one embodiment, the location of the patient relative to the CGCI system (including the radar system 1000) can be determined by using the radar 1000 to locate a plurality of fiduciary markers. In one embodiment, the data from the radar 1000 is used to locate the body with respect to an imaging system. The catheter position data from the radar 1000 can be superimposed (synchronized) with the images produced by the imaging system. The ability of the radar and the optional Hall effect sensors 350 to accurately position the catheter tip 377 relative to the stereotactic frame allows the pole pieces to be moved by the actuators 109, 140 to optimize the location of the magnet poles with respect to the patient 390 and thus reduce the power needed to manipulate the catheter tip.

FIGS. 8A and 8B shows one embodiment of a catheter assembly 375 and guidewire assembly 379 to be used with the CGCI apparatus 1500. The catheter assembly 375 is a tubular tool that includes a catheter body 376 which extends into a flexible section 378 that possesses sufficient flexibility for allowing a relatively more rigid responsive tip 377 to be steered through the patient. The tip 377 can be replaced by the tip 21 of FIG. 3, the tip 61 of FIG. 4, or the tip 81 of FIG. 5.

In one embodiment, the magnetic catheter assembly 375 in combination with the CGCI apparatus 1500 reduces or eliminates the need for the plethora of shapes normally needed to perform diagnostic and therapeutic procedures. During a conventional catheterization procedure, the surgeon often encounters difficulty in guiding the conventional catheter to the desired position, since the process is manual and relies on manual dexterity to maneuver the catheter through a tortuous path of, for example, the cardiovascular system. Thus, a plethora of catheters in varying sizes and shapes are to be made available to the surgeon in order to assist him/her in the task, since such, tasks require different bends in different situations due to natural anatomical variations within and between patients.

By using the CGCI apparatus 1500, only a single catheter is needed for most, if not all patients. The catheterization procedure is now achieved with the help of the CGCI system 1500 that guides the magnetic catheter and guidewire assembly 375 and 379 to the desired position within the patient's body 390 as dictated by the surgeon's manipulation of the virtual tip 905. The magnetic catheter and guidewire assembly 375, 379 (i.e. the magnetic tip 377 can be attracted or repelled by the electromagnets of the CGCI apparatus 1500) provides the flexibility needed to overcome tortuous paths, since the CGCI apparatus 1500 overcomes most, if not all the physical limitations faced by the surgeon while attempting to manually advance the catheter tip 377 through the patient's body.

In one embodiment, the catheter tip 377 includes a guidewire assembly 379, a guidewire body 380 and a tip 381 response to magnetic fields. The tip 377 is steered around sharp bends so as to navigate a torturous path. The responsive tips 377 and 381 of both the catheter assembly 375 and the guidewire assembly 379, respectively, include magnetic elements such as permanent magnets. The tips 377 and 381 include permanent magnets that respond to the external flux generated by the electromagnets 110, 115, 116 and its symmetric counterpart 100.

In one embodiment, the responsive tip 377 of the catheter assembly 375 is tubular, and the responsive tip 381 of the guidewire assembly 379 is a solid cylinder. The responsive tip 377 of the catheter assembly 375 is a dipole with longitudinal polar orientation created by the two ends of the magnetic element positioned longitudinally within it. The responsive tip 381 of the guidewire assembly 379 is a dipole with longitudinal polar orientation created by two ends of the magnetic element 377 positioned longitudinally within it. These longitudinal dipoles allow the manipulation of both responsive tip 377 and 381 with the CGCI apparatus 1500, as the electromagnet assemblies 100, 101, and will act on the tips 377 and 381 and "drag" them in unison to a desired position as dictated by the operator.

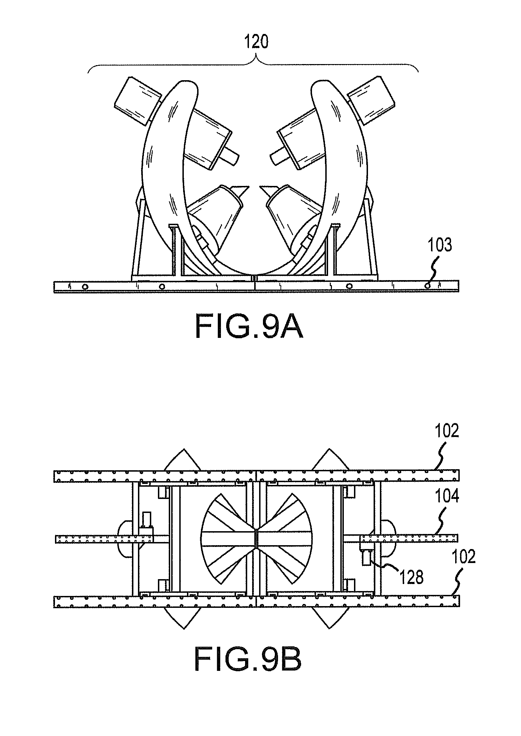

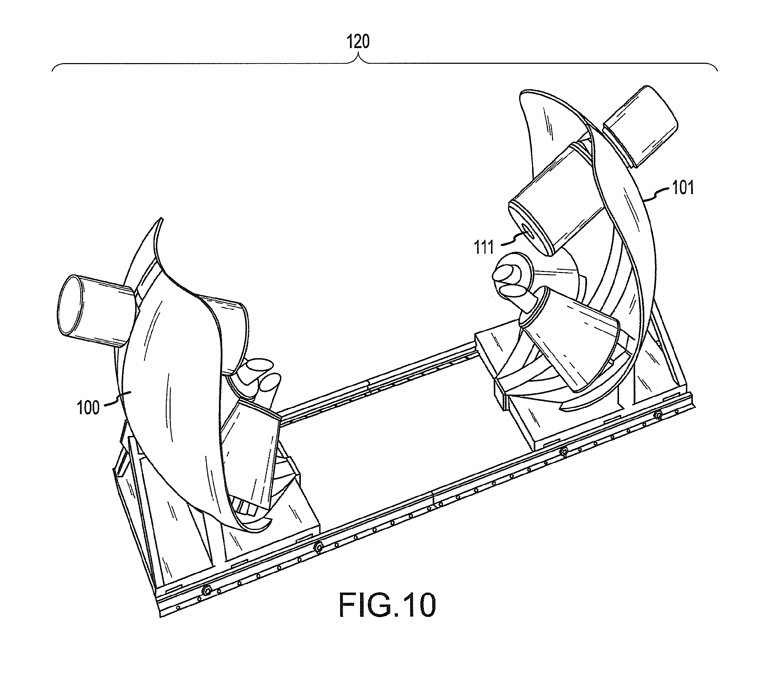

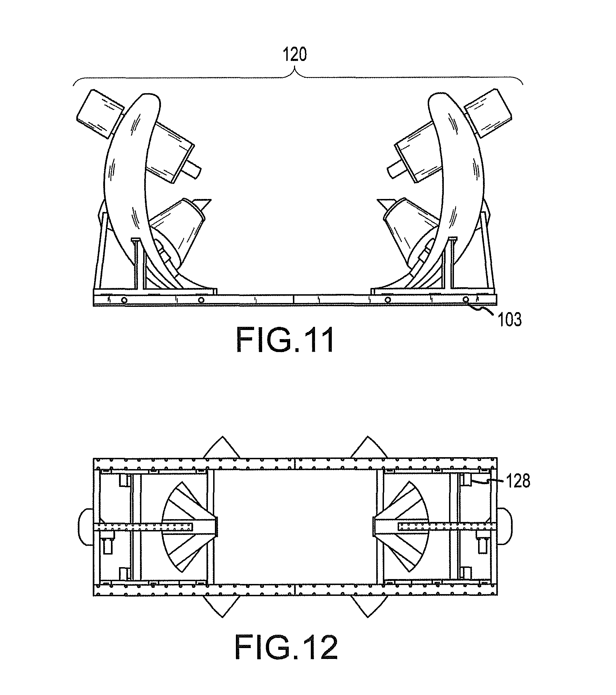

FIGS. 9A and 9B show additional views of the CGCI structural support assembly 120. The structural support assembly 120 is configured so as to facilitate the use of X-Ray and/or other surgical medical equipment 502 in and around the patient during operation. The two symmetrical left 100 and right 101 electromagnetic clusters are mounted on the stainless steel guide rails 102, allowing the two sections 100 and 101 to move away from each other as shown in FIGS. 10-12. The rails 102 are bolted to a floor or mounting pad. The cluster on the CGCI structure 120 rolls inside the rails 102, under relatively tight tolerance to prevent lateral or vertical movement during a seismic event. In one embodiment, the rails 102 are designed to withstand the forces of a Zone 4 seismic event without allowing the CGCI structure to escape containment.

A stainless steel spur toothed rail 104 is bolted to the floor or mounting pad under the CGCI structure 120. A Servo Dynamic model HJ96 C-44 brushless servomotor 128 (max 27 lb.-in torque) with its associated servomotor amplifier model 815-BL 129 are provided to move the clusters 101, 100. The motor has a reduction gearbox with a ratio of 100:1. A stainless steel spur gear attached to the reduction gear shaft meshes with the spur toothed rail 104. The propulsion system 150 is configured to exert up to 2700 lbs. of force to move the CGCI sections 100 and 101.

FIGS. 9A and 9B further show the CGCI assembly 120 when the system is set in the "operational mode." The two symmetrical clusters 100 and 101 are engaged as described above. FIGS. 9A and 9B show the location of the spur toothed rail 104 and the brushless servo motor 128.

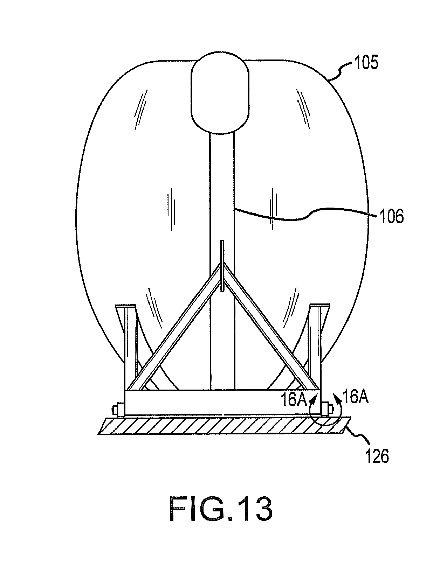

FIGS. 10-13 are isometric views of the CGCI assembly 120 when its main two symmetric left 100 and right 101 coil clusters are in a fully open mode (non operational) and the magnetic cores are retracted. The rear view of the symmetrical one half of the CGCI shows the parabolic flux collector shields 105 with the C-Arm upper cylinder coil support 106. In one embodiment, the CGCI assembly 120 is configured to meet the structural as well as safety considerations associated with the generation of a magnetic field of 2 Tesla.

FIG. 14 depicts the CGCI system 1500 top architecture showing the major elements comprising the controller 501 of the magnetic circuit. The controller 501 includes a system memory, a torque/force matrix algorithm residing in 528 and a CPU/computer 527. The CPU/computer such as PC 527 provides computation and regulation tasks. FIG. 14 further shows the six-coil electromagnetic circuit formed out of coils 51A, 51 B, 51C, 51D, 51 AT and 51 DT and the magnetic field sensors (MFS) 351, 352, 353, 354, 355 and 356 such as Hall sensor ring 350 mounted on an assembly forming the X, Y, and Z axis controls. A D/A converter 550 and an I/O block 551 provide communication between the controller 501 and the coils 51A and the hydraulic systems 140. The six channel DC amplifier 525 provides current to the coils.

FIG. 14 shows the relationship and command structure between the joystick 900, the virtual tip 905, and the CPU 701. The CPU 701 displays control conveying real time images generated by the X-ray, radar 1000, or other medical imaging technologies such as fluoroscopy, MRI, PET SCAN, CAT SCAN, etc., on a display 730. A flow diagram of the command structure of the control scheme is shown by the use of the 2D virtual plane coil polarity matrixes. By assigning the coil position and polarity elements to the directions of torque rotation and force field gradient on each 2D plane of a six coil cluster 414, a computer program such as MathLab or Math Cad is able to sift through the combination matrixes and compute the proper combination for the six coil current polarities and amplitudes. In one embodiment, a boundary condition controller is used for regulating the field strength 405 and field gradient 406 in the effective region. The controller 501 computes the fields in the neighborhood of the catheter tip 377 and as defined by the fields on the 2D planes in the effective area. Rules for computing the fields with rotated coil on the surface of the sphere are set forth in US 2007/0016066.

In one embodiment, look-up tables are used as a reference library for use by the controller 501. Lookup tables of the setting of various scenarios of force as well as torque position and magnitude allow the controller 501 to use a learning algorithm for the control computations. The look-up tables shorten the computational process for optimal configuration and setting of the coil currents and pole positions. The D/A and A/D system 550 allows the connection of voltage and current measuring instruments as well as input from the magnetic field sensor (MFS) 350 array, the MFS 351, 352, 353, 354, 355 and 356. The magnetic field sensor measuring the boundary plane field strength allows the CGCI to use a low-level logic algorithm to compute the positions, settings, coil currents, etc. The low-level simulation is performed prior to activating the power section of the CGCI apparatus 1500, thus providing a "soft" level check prior to action performed by actual machine. The two-level control architecture that starts with low-level simulation architecture of low-level simulation allows the surgeon or operator of the CGCI apparatus 1500 to test each movement prior to actually performing the move. US 2007/0016066 describes the field regulator loop outlined in FIG. 14 using the Hall effect ring 350.

Instead of using the radar system to identify the position of the catheter tip 377, the present invention may rely on the use of the monitoring or measuring electrodes (58, 59 in FIG. 3; 80 in FIGS. 4 and 5), optionally in conjunction with a visualization and mapping tool such as the EnSite NavX™ technology available from St. Jude Medical, Inc. See, e.g., U.S. Patent Nos. 6,990,370 and 6,939,309, the entire disclosures of which are incorporated herein by reference.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the scope of the invention as defined in the appended claims.

## Claims

1. An irrigated ablation electrode assembly (10, 11) for use with an irrigated catheter device (12), the irrigated ablation electrode assembly (10, 11) comprising:
at least one passageway (24) for a fluid with an outlet (22) disposed at an external surface of the electrode assembly;
a permanent magnet (48);
a shield (52, 56), the shield (52, 56) being substantially less oxidizable than the permanenet magnet (48); and
an electrode (50, 52) having an external electrode surface,
wherein the electrode (50, 52) is disposed at a distal portion (18) of the electrode assembly, and wherein the electrode assembly (10, 11) further comprises a proximal portion (20), and the permanent magnet (48) is disposed in the distal portion (18),
**characterized in that** the proximal portion includes the at least one passageway (24) for a fluid with the outlet (22) disposed at an external surface of the proximal portion (20), and
the shield (52, 56) separates the permanent magnet (48) from the at least one passageway (24) and from an exterior.

2. The irrigated ablation electrode assembly (10, 11) of claim 1, wherein the electrode (50, 52) forms at least a portion of the shield (52, 56), and wherein the electrode comprises an electrically conductive material that is substantially less oxidizable than the permanent magnet (48),
wherein preferably the electrically conductive material is selected from the group consisting of platinum, gold, tantalum, iridium, stainless steel, palladium, and mixtures thereof, and wherein further preferably the electrically conductive material is plated onto a substrate made of a biocompatible material that is substantially less oxidizable than the permanent magnet (48).

3. The irrigated ablation electrode assembly (10, 11) of any one of the preceding claims, wherein the shield (52, 56) comprises one or more materials selected from the group consisting of silicone, polyimide, platinum, gold, tantalum, iridium, stainless steel, palladium, and mixtures thereof.

4. The irrigated ablation electrode assembly (10, 11) of any one of the preceding claims, wherein the permanent magnet (48) comprises NdFeB.

5. The irrigated ablation electrode assembly (10, 11) of any one of the preceding claims further comprising at least one mapping electrode (58, 59) spaced proximally from the electrode (50, 52) which is a distal electrode capable of ablation.

6. The irrigated ablation electrode assembly (10, 11) of any one of the preceding claims, wherein the proximal portion (20) comprises a material which is electrically nonconductive and has a lower thermal conductivity than a material of the electrode (50, 52).

7. The irrigated ablation electrode assembly (10, 11) of any one of the preceding claims, wherein the at least one passageway (24) extends toward the electrode (50, 52) at an acute angle with respect to the longitudinal axis of the proximal portion (20).

8. The irrigated ablation electrode assembly (10, 11) of any one of the preceding claims, wherein the proximal portion (20) comprises a material which is electrically nonconductive, and wherein the external surface of the proximal portion (20) and the external electrode surface of the electrode (50, 52) at the distal portion meet at an intersection, and wherein the at least one proximal passageway (24) is configured to direct a fluid flow through the outlet toward a region adjacent the intersection.

9. The irrigated ablation electrode assembly (10, 11) of any one of the preceding claims, wherein the electrode assembly further comprises at least one temperature sensor (26) disposed in the permanent magnet (48).

10. A catheter (12) comprising:
a shaft; and
an irrigated ablation electrode assembly (10, 11) according to any one of the preceding claims and coupled to a distal end of the shaft.

11. The catheter (12) of claim 10 further comprising a second permanent magnet disposed near the distal end of the shaft and spaced from the permanent magnet (48) in the irrigated ablation electrode assembly (10, 11).

## Patentansprüche

1. Irrigations-Ablations-Elektrodenanordnung (10, 11) zur Verwendung mit einer Irrigations-Kathetervorrichtung (12), wobei die Irrigations-Ablations-Elektrodenanordnung (10, 11) aufweist:
zumindest einen Durchgang (24) für ein Fluid mit einem Auslass (22), der an einer äußeren Oberfläche der Elektrodenanordnung angeordnet ist,
einen Permanentmagneten (48),
eine Abschirmung (52, 56), wobei die Abschirmung (52, 56) im Wesentlichen weniger oxidierbar ist als der Permanentmagnet (48), und
eine Elektrode (50, 52), die eine äußere Elektrodenoberfläche aufweist,
wobei die Elektrode (50, 52) an einem distalen Teil (18) der Elektrodenanordnung angeordnet ist, und wobei die Elektrodenbauanordnung (10, 11) weiter einen proximalen Teil (20) aufweist, und der Permanentmagnet (48) in dem distalen Teil (18) angeordnet ist,
**dadurch gekennzeichnet, dass** der proximale Teil den zumindest einen Durchgang (24) für ein Fluid mit dem Auslass (22), der an einer äußeren Oberfläche des proximalen Teils (20) angeordnet ist, enthält, und
die Abschirmung (52, 56) den Permanentmagneten (48) von dem zumindest einen Durchgang (24) und von einer Außenseite trennt.

2. Irrigations-Ablations-Elektrodenanordnung (10, 11) nach Anspruch 1, bei dem die Elektrode (50, 52) zumindest einen Teil der Abschirmung (52, 56) ausbildet, und wobei die Elektrode ein elektrisch leitendes Material aufweist, das im Wesentlichen weniger oxidierbar als der Permanentmagnet (48) ist,
bei dem bevorzugt das elektrisch leitende Material aus der Gruppe gewählt ist, die aus Platin, Gold, Tantal, Iridium, Edelstahl, Palladium und Mischungen davon besteht, und wobei weiter bevorzugt das elektrisch leitende Material auf ein Substrat, das aus einem biologisch verträglichem Material hergestellt ist, das im Wesentlichen weniger oxidierbar als der Permanentmagnet (48) ist, plattiert ist.

3. Irrigations-Ablations-Elektrodenanordnung (10, 11) nach einem der vorhergehenden Ansprüche, wobei die Abschirmung (52, 56) eine oder mehrere Materialien aufweist, die aus der Gruppe gewählt sind, die aus Silikon, Polyimid, Platin, Gold, Tantal, Iridium, Edelstahl, Palladium und Mischungen davon besteht.

4. Irrigations-Ablations-Elektrodenanordnung (10, 11) nach einem der vorhergehenden Ansprüche, bei dem der Permanentmagnet (48) NdFeB aufweist.

5. Irrigations-Ablations-Elektrodenanordnung (10, 11) nach einem der vorhergehenden Ansprüche, die weiter zumindest eine Abbildungselektrode (58, 59), die von der Elektrode (50, 52), die eine distale Elektrode ist und für eine Ablation geeignet ist, proximal beabstandet ist.

6. Irrigations-Ablations-Elektrodenanordnung (10, 11) nach einem der vorhergehenden Ansprüche, wobei der proximale Teil (20) ein Material aufweist, das nicht elektrisch leitend ist und eine geringere Wärmeleitfähigkeit als ein Material der Elektrode (50, 52) aufweist.

7. Irrigations-Ablations-Elektrodenanordnung (10, 11) nach einem der vorhergehenden Ansprüche, wobei sich der zumindest eine Durchgang (24) in Richtung der Elektrode (50, 52) mit einem spitzen Winkel in Bezug auf die Längsachse des proximalen Teils (20) erstreckt.

8. Irrigations-Ablations-Elektrodenanordnung (10, 11) nach einem der vorhergehenden Ansprüche, wobei der proximale Teil (20) ein Material aufweist, das nicht elektrisch leitend ist, und wobei die äußere Oberfläche des proximalen Teils (20) und die äußere Elektrodenoberfläche der Elektrode (50, 52) an dem distalen Teil an einer Schnittfläche zusammentreffen, und wobei der zumindest eine proximale Durchgang (24) dazu konfiguriert ist, ein Fluid, das durch den Auslass in Richtung eines Bereichs angrenzend zu der Schnittfläche strömt, zu richten.

9. Irrigations-Ablations-Elektrodenanordnung (10, 11) nach einem der vorhergehenden Ansprüche, wobei die Elektrodenanordnung weiter zumindest einen Temperatursensor (26), der in dem Permanentmagneten (48) angeordnet ist, aufweist.

10. Katheter (12), mit
einem Schaft, und
einer Irrigations-Ablations-Elektrodenanordnung (10, 11) nach einem der vorhergehenden Ansprüche und die an ein distales Ende des Schaftes gekoppelt ist.

11. Katheter (12) nach Anspruch 10, der weiter einen zweiten Permanentmagneten aufweist, der in der Nähe des distalen Endes des Schaftes angeordnet ist und von dem Permanentmagneten (48) in der Irrigations-Ablations-Elektrodenanordnung (10, 11) beabstandet ist.

## Revendications

1. Ensemble électrode d'ablation irriguée (10, 11), pour une utilisation avec un dispositif (12) de cathéter irrigué, l'ensemble électrode d'ablation irriguée (10,11) comprenant :
au moins un passage (24) pour un fluide avec un orifice de sortie (22) disposé au niveau d'une surface externe de l'ensemble électrode ;
un aimant permanent (48) ;
une protection (52, 56), la protection (52, 56) étant sensiblement moins oxydable que l'aimant permanent (48) ; et
une électrode (50, 52) ayant une surface d'électrode externe,
dans lequel l'électrode (50, 52) est disposé à une portion distale (18) de l'ensemble électrode, et dans lequel l'ensemble électrode (10, 11) comprend en outre une portion proximale (20), et l'aimant permanent (48) est disposé dans la portion distale (18),
**caractérisé en ce que** la portion proximale inclut le au moins un passage (24) pour un fluide avec l'orifice de sortie (22) disposé au niveau d'une surface externe de la portion proximale (20), et
la protection (52, 56) sépare l'aimant permanent (48) du au moins un passage (24) et d'un extérieur.

2. L'ensemble électrode d'ablation irriguée (10, 11) selon la revendication 1, dans lequel l'électrode (50, 52) forme au moins une portion de la protection (52, 56), et dans lequel l'électrode comprend un matériau électriquement conducteur qui est sensiblement moins oxydable que l'aimant permanent (48),
dans lequel, avantageusement, le matériau électriquement conducteur est sélectionné à partir du groupe constitué de platine, d'or, de tantale, d'iridium, d'acier inoxydable, de palladium, et de mélanges de ceux-là, et dans lequel en outre, avantageusement, le matériau électriquement conducteur est plaqué sur un substrat fait de matériau biocompatible qui est sensiblement moins oxydable que l'aimant permanent (48).

3. L'ensemble électrode d'ablation irriguée (10, 11) selon l'une quelconque des revendications précédentes, dans lequel la protection (52, 56) comprend un ou plusieurs matériaux sélectionnés à partir d'un groupe constitué de silicone, de polyimide, de platine, d'or, de tantale, d'iridium, d'acier inoxydable, de palladium, et de mélanges de ceux-là.

4. L'ensemble électrode d'ablation irriguée (10, 11) selon l'une quelconque des revendications précédentes, dans lequel l'aimant permanent (48) comprend du NdFeB.

5. L'ensemble électrode d'ablation irriguée (10, 11) selon l'une quelconque des revendications précédentes, comprend en outre au moins une électrode de cartographie (58, 59) espacée de manière proximale de l'électrode (50, 52) qui est une électrode distale capable d'ablation.

6. L'ensemble électrode d'ablation irriguée (10, 11) selon l'une quelconque des revendications précédentes, dans lequel la portion proximale (20) comprend un matériau qui est électriquement non conducteur et présente une conductivité thermique plus basse qu'un matériau de l'électrode (50, 52).

7. L'ensemble électrode d'ablation irriguée (10, 11) selon l'une quelconque des revendications précédentes, dans lequel le au moins un passage (24) s'étend vers l'électrode (50, 52) avec un angle aigu par rapport à l'axe longitudinal de la portion proximale (20).

8. L'ensemble électrode d'ablation irriguée (10, 11) selon l'une quelconque des revendications précédentes, dans lequel la portion proximale (20) comprend un matériau qui est électriquement non conducteur, et dans lequel la surface externe de la portion proximale (20) et la surface d'électrode externe de l'électrode (50, 52) au niveau de la portion distale se rencontrent en une intersection, et dans lequel le au moins un passage (24) est configuré pour diriger un écoulement de fluide à travers l'orifice de sortie vers une région adjacente à l'intersection.

9. L'ensemble électrode d'ablation irriguée (10, 11) selon l'une quelconque des revendications précédentes, dans lequel l'ensemble électrode comprend en outre au moins un capteur de température (26) disposé dans l'aimant permanent (48).

10. Un cathéter (12) comprenant :
un arbre ; et
un ensemble électrode d'ablation irriguée (10, 11) selon l'une quelconque des revendications précédentes et couplée à une extrémité distale de l'arbre.

11. Le cathéter (12) selon la revendication 10 comprenant en outre un second aimant permanent disposé à proximité de l'extrémité distale et espacée de l'aimant permanent (48) dans l'ensemble électrode d'ablation irriguée (10, 11).
